# EUROPÄISCHE PATENTANMELDUNG

(11) **EP 4 657 454 A1**
(43) Veröffentlichungstag der Anmeldung: **03.12.2025**
(21) Anmeldenummer: 25178796.6
(22) Anmeldetag: 26.05.2025
(51) Int. Cl.: G16H 40/60

(54) **SCHÄTZEN EINER KONZENTRATION EINES ATEMGASES IM BLUT EINES PATIENTEN**

(30) Priorität: 27.05.2024 DE 102024114793
(71) Anmelder: Löwenstein Medical Technology S.A., 2557 Luxembourg (LU)
(72) Erfinder: Kremeier, Peter, 76149 Karlsruhe (DE); Tusman, Gerardo, 7600 Mar del Plata (AR)
(74) Vertreter: Löwenstein Medical IP

(57) **Zusammenfassung**

Ein Verfahren zum Schätzen einer Konzentration eines Atemgases im Blut eines Patienten umfasst: Empfangen von Messdaten, die einen volumenabhängigen Verlauf einer Konzentration des Atemgases in einem vom Patienten ausgeatmeten Atemluftstrom abhängig von einem vom Patienten ausgeatmeten Atemluftvolumen anzeigen; Erzeugen von Eingabedaten (14) aus den Messdaten, wobei die Eingabedaten (14) eine Matrix von Werten für verschiedene Parameter bezüglich des volumenabhängigen Verlaufs umfassen; Eingeben der Eingabedaten (14) in ein Machine-Learning-Modul (15), das trainiert wurde, um die Eingabedaten (14) in Ausgabedaten (17) umzuwandeln, wobei die Ausgabedaten (17) eine Konzentration des Atemgases im Blut des Patienten anzeigen; Ausgeben der Ausgabedaten (17) durch das Machine-Learning-Modul (15).

## Beschreibung

### Technisches Gebiet

Die Erfindung betrifft ein Verfahren zum Schätzen einer Konzentration eines Atemgases im Blut eines Patienten. Des Weiteren betrifft die Erfindung ein Verfahren zum Trainieren eines Machine-Learning-Moduls zur Verwendung in einem solchen Verfahren. Ferner betrifft die Erfindung eine Datenverarbeitungsvorrichtung, ein Computerprogramm und ein computerlesbares Medium zum Ausführen von mindestens einem dieser Verfahren sowie ein medizintechnisches Gerät.

### Stand der Technik

Bei der maschinellen Beatmung anästhesierter und kritisch kranker Patienten muss der ordnungsgemäße Austausch biologischer Gase kontinuierlich aufrechterhalten werden. Den Goldstandard für die Beurteilung des Gasaustauschs im klinischen Bereich stellt die Analyse biologischer Gase in arteriellen Blutproben dar. Dabei werden die Partialdrücke von Kohlendioxid und Sauerstoff im Blut mit dem Anteil des eingeatmeten Sauerstoffs und der alveolären Ventilation abgeglichen, um festzustellen, ob ein Atemversagen vorliegt oder nicht. Eine solche Blutgasanalyse ist jedoch invasiv und zeitaufwendig.

Daneben ist es möglich, den alveolären Kohlendioxidpartialdruck (p_{A}CO₂) mittels Kapnographie nicht invasiv zu messen. Eine solche Messung kann jedoch eine herkömmliche Blutgasanalyse - insbesondere bei anästhesierten und kritisch kranken Patienten - bislang nicht ersetzen.

### Offenbarung der Erfindung

Eine Aufgabe der Erfindung kann darin gesehen werden, ein Verfahren zu schaffen, das es ermöglicht, eine Konzentration eines Atemgases im Blut eines Patienten nicht invasiv mit hinreichender Genauigkeit zu bestimmen. Eine weitere Aufgabe der Erfindung kann darin gesehen werden, ein Verfahren zum Trainieren eines entsprechenden Machine-Learning-Moduls, eine entsprechende Datenverarbeitungsvorrichtung, ein entsprechendes Computerprogramm, ein entsprechendes computerlesbares Medium sowie ein entsprechendes medizintechnisches Gerät bereitzustellen.

Diese Aufgaben werden durch die Gegenstände der unabhängigen Ansprüche gelöst. Vorteilhafte Ausführungsformen der Erfindung sind in den abhängigen Ansprüchen, der nachfolgenden Beschreibung und den beigefügten Figuren dargelegt.

Ein erster Aspekt der Erfindung betrifft ein computerimplementiertes Verfahren zum Schätzen einer Konzentration eines Atemgases im Blut eines Patienten. Das Verfahren umfasst: Empfangen von Messdaten, die einen volumenabhängigen Verlauf einer Konzentration des Atemgases in einem vom Patienten ausgeatmeten Atemluftstrom abhängig von einem vom Patienten ausgeatmeten Atemluftvolumen anzeigen; Erzeugen von Eingabedaten aus den Messdaten, wobei die Eingabedaten eine Matrix von Werten für verschiedene Parameter bezüglich des volumenabhängigen Verlaufs umfassen; Eingeben der Eingabedaten in ein Machine-Learning-Modul, das trainiert wurde, um die Eingabedaten in Ausgabedaten umzuwandeln, wobei die Ausgabedaten eine Konzentration des Atemgases im Blut des Patienten anzeigen; Ausgeben der Ausgabedaten durch das Machine-Learning-Modul.

Ein zweiter Aspekt der Erfindung betrifft ein computerimplementiertes Verfahren zum Trainieren eines Machine-Learning-Moduls für ein medizintechnisches Gerät. Das Verfahren umfasst: Empfangen mehrerer Messdatensätze, die jeweils Messdaten umfassen, die einen volumenabhängigen Verlauf einer Konzentration eines Atemgases in einem von einem Patienten ausgeatmeten Atemluftstrom abhängig von einem vom Patienten ausgeatmeten Atemluftvolumen anzeigen, wobei die Messdaten verschiedener Messdatensätze zumindest teilweise verschiedenen Patienten zugeordnet sind; Erzeugen mehrerer Trainingsdatensätze aus den Messdatensätzen, wobei jeder Trainingsdatensatz einem der Patienten zugeordnet ist und eine Matrix von Werten für verschiedene Parameter bezüglich des volumenabhängigen Verlaufs umfasst; Eingeben eines jeden Trainingsdatensatzes als Eingabedaten in das Machine-Learning-Modul, das konfiguriert ist, um die Eingabedaten in Ausgabedaten umzuwandeln, wobei die Ausgabedaten eine Konzentration des Atemgases im Blut des jeweiligen Patienten anzeigen; Ausgeben der Ausgabedaten durch das Machine-Learning-Modul; Bestimmen einer Abweichung der Ausgabedaten von Zieldaten, die dem jeweiligen (den Ausgabedaten zugrunde liegenden) Trainingsdatensatz zugeordnet sind; Anpassen von Gewichten des Machine-Learning-Moduls in einem Optimierungsverfahren, um die Abweichung zu verringern.

Es ist möglich, dass die Verfahren automatisch durch einen Prozessor, beispielsweise durch einen Prozessor eines medizintechnischen Geräts, ausgeführt werden. Das in dem Verfahren gemäß dem ersten Aspekt der Erfindung verwendete Machine-Learning-Modul kann mit dem Verfahren gemäß dem zweiten Aspekt der Erfindung trainiert worden sein. Das Verfahren gemäß dem ersten Aspekt der Erfindung kann zusätzlich die Schritte des Verfahrens gemäß dem zweiten Aspekt der Erfindung umfassen.

Der hier vorgestellte Ansatz beruht auf der Erkenntnis, dass zur Schätzung einer Konzentration eines Atemgases im Blut eines Patienten, insbesondere in dessen arteriellem Blut, die Kurve eines volumetrischen Kapnogramms oder Oxigramms mithilfe eines Machine-Learning-Algorithmus analysiert werden kann. Diese Kurve ändert sich abhängig von Schwankungen in der Lungenventilation und -perfusion des Patienten. Solche Schwankungen können bei einer Schätzung mit herkömmlichen Methoden zu größeren Ungenauigkeiten führen.

Hingegen ermöglichen die vor- und nachstehend beschriebenen Verfahren auch bei verschiedenen Patienten und/oder bei starken Schwankungen der Lungenfunktion eine sehr genaue Schätzung der Konzentration des Atemgases im Blut des Patienten. Dies hat den Vorteil, dass eine invasive Blutgasanalyse weniger häufig durchgeführt werden muss oder sogar entfallen kann.

Im Vergleich zu einer Ausführungsform, bei der die Konzentration des Atemgases im Blut des Patienten unmittelbar anhand der (rohen) Messdaten geschätzt wird oder bei der die (rohen) Messdaten als die Eingabedaten verwendet werden, haben die vor- und nachstehend beschriebenen Verfahren zudem den Vorteil, dass deutlich weniger Rechenleistung erforderlich ist und auch bei weniger guten Messdaten eine ausreichend genaue Schätzung ermöglicht wird. Zudem wird das Risiko von Fehlinterpretationen reduziert, weil die Eingabedaten im Gegensatz zu (rohen) Messdaten vordefiniert sind.

Besonders vorteilhaft ist es, wenn mehrere Parameter bezüglich der Kurve analysiert werden. Ein solcher multivariater Ansatz liefert mehr Informationen für die Schätzung, als wenn nur ein einzelner Parameter, beispielsweise der alveoläre Partialdruck des betreffenden Atemgases, analysiert wird, und ermöglicht in Kombination mit einem entsprechend konfigurierten Machine-Learning-Algorithmus eine deutlich genauere und robustere Schätzung.

Nachfolgend werden einige Begriffe näher erläutert.

Unter "Patient" kann ein Beatmungspatient verstanden werden, d. h. ein menschliches oder tierisches Subjekt, das mittels eines Beatmungsgeräts beatmet wird oder werden soll.

Unter "Atemgas" kann beispielsweise eines der folgenden Gase verstanden werden: Kohlendioxid, Sauerstoff, Stickstoff, Wasserdampf, Narkosegas.

Unter "Atemluft" wie in "Atemluftstrom" oder "Atemluftvolumen" kann ein das Atemgas umfassendes Atemgasgemisch verstanden werden.

Unter "Konzentration" kann allgemein ein Anteil oder eine Menge, insbesondere ein Partialdruck, verstanden werden.

Bei der Konzentration des Atemgases im Blut des Patienten kann es sich beispielsweise um einen arteriellen und/oder venösen Partialdruck des Atemgases handeln.

Die Messdaten können zumindest teilweise unter Verwendung einer entsprechenden Sensorik, beispielsweise einer Sensorik eines medizintechnischen Geräts, erzeugt worden sein. Beispielsweise können die Messdaten nicht invasiv durch Kapnographie und/oder Oxigraphie erzeugt worden sein. Anders ausgedrückt kann es sich bei den Messdaten beispielsweise um Daten aus einem Kapnogramm und/oder einem Oxigramm handeln.

Die Messdaten, die den Trainingsdatensätzen zugrunde liegen, können reale (d. h. aus einer realen Messung resultierende) Daten und/oder simulierte Daten umfassen. Die simulierten Daten können - im Gegensatz zu den realen Daten - unter Verwendung einer Simulationsumgebung, in der Lungenzustände verschiedener Patienten durch einen Computer simuliert werden, erzeugt worden sein.

Unter "Eingabedaten" können von den Messdaten abweichende und/oder im Gegensatz zu den Messdaten speziell an das Machine-Learning-Modul angepasste Daten verstanden werden. Insbesondere können die Eingabedaten gegenüber den Messdaten komprimierte Daten sein. Es ist möglich, dass die Messdaten in ein weiteres Machine-Learning-Modul eingegeben werden, das trainiert wurde, um die Messdaten in die Eingabedaten umzuwandeln.

Unter "Machine-Learning-Modul" kann ein Hard- und/oder Softwaremodul zum Umwandeln von Eingabedaten in Ausgabedaten in einem durch maschinelles Lernen parametrierten, d. h. trainierten Algorithmus verstanden werden. Ein solcher Algorithmus kann beispielsweise ein künstliches neuronales Netz, ein Entscheidungsbaum, ein Random Forest, ein k-nächste-Nachbarn-Algorithmus, eine Support-Vector-Maschine, ein Bayes-Klassifikator, ein k-Means-Algorithmus, ein genetischer Algorithmus, ein Kernelregressionsalgorithmus, ein Diskriminanzanalyse-Algorithmus oder eine Kombination von mindestens zwei dieser Beispiele sein.

Jedem Trainingsdatensatz, der in das Machine-Learning-Modul eingegeben wird, kann ein Satz vordefinierter Zieldaten zugeordnet sein. Die Zieldaten können beispielsweise einen dem jeweiligen Trainingsdatensatz zugeordneten Zielwert für die Konzentration des Atemgases im Blut des jeweiligen Patienten umfassen. Insbesondere können die Zieldaten ein Ergebnis einer Messung (z. B. einer Blutgasanalyse) anzeigen, die zum gleichen Zeitpunkt und/oder kurz vor und/oder kurz nach dem Zeitpunkt durchgeführt wurde, zu dem die dem jeweiligen Trainingsdatensatz zugrunde liegenden Messdaten erzeugt wurden. Die Zieldaten können reale (d. h. aus einer realen Messung resultierende) Daten und/oder simulierte Daten umfassen. Die simulierten Daten können - im Gegensatz zu den realen Daten - unter Verwendung einer Simulationsumgebung, in der Lungenzustände verschiedener Patienten durch einen Computer simuliert werden, zusammen mit dem jeweiligen Trainingsdatensatz erzeugt worden sein.

Zum Bestimmen der Abweichung der Ausgabedaten von den Zieldaten können die Ausgabedaten und die Zieldaten in eine geeignete Verlustfunktion zum Berechnen eines die Abweichung quantifizierenden Scores eingegeben werden. Der Score kann beispielsweise mithilfe der Methode der kleinsten Quadrate berechnet werden.

Unter "Optimierungsverfahren" kann ein iteratives Verfahren zum Minimieren der Verlustfunktion verstanden werden, beispielsweise ein Gradientenverfahren, insbesondere ein stochastisches Gradientenverfahren, mit Backpropagation.

Ein dritter Aspekt der Erfindung betrifft eine Datenverarbeitungsvorrichtung. Die Datenverarbeitungsvorrichtung umfasst einen Prozessor, der konfiguriert ist, um mindestens eines der vor- und nachstehend beschriebenen Verfahren auszuführen.

Unter "Datenverarbeitungsvorrichtung" kann allgemein ein Computer verstanden werden. Die Datenverarbeitungsvorrichtung kann Hard- und/oder Softwarekomponenten umfassen. Die Datenverarbeitungsvorrichtung kann beispielsweise ein Steuergerät, ein PC, ein Server, ein Laptop, ein Tablet, ein Smartphone oder eine Kombination von mindestens zwei dieser Beispiele sein. Alternativ kann unter "Datenverarbeitungsvorrichtung" mindestens eine Hard- und/oder Softwarekomponente von mindestens einem dieser Beispiele verstanden werden.

Unter "Prozessor" kann beispielsweise eine CPU *(central processing unit),* ein Grafikprozessor, eine TPU *(tensor processing unit)* oder eine Kombination von mindestens zwei dieser Beispiele verstanden werden.

Zusätzlich zum Prozessor kann die Datenverarbeitungsvorrichtung mindestens eine der folgenden Komponenten umfassen: einen Speicher, ein Bussystem zur Datenkommunikation zwischen dem Prozessor und dem Speicher, eine Datenkommunikationsschnittstelle zur drahtlosen und/oder drahtgebundenen Datenkommunikation mit Peripheriegeräten.

Es wird darauf hingewiesen, dass Merkmale der vor- und nachstehend beschriebenen Verfahren auch Merkmale der Datenverarbeitungsvorrichtung sein können (und umgekehrt).

Ein vierter Aspekt der Erfindung betrifft ein medizintechnisches Gerät. Das medizintechnische Gerät umfasst eine Sensorik zum Erzeugen von Messdaten, die einen volumenabhängigen Verlauf einer Konzentration eines Atemgases in einem von einem Patienten ausgeatmeten Atemluftstrom abhängig von einem vom Patienten ausgeatmeten Atemluftvolumen anzeigen, sowie eine Datenverarbeitungsvorrichtung, wie sie vor- und nachstehend beschrieben wird.

Bei dem medizintechnischen Gerät kann es sich beispielsweise um ein Beatmungsgerät zum invasiven und/oder nicht invasiven Beatmen eines Patienten und/oder um einen Überwachungsmonitor zum Überwachen von Vitalparametern eines Patienten handeln.

Die Sensorik kann einen oder mehrere Gassensoren umfassen. Unter "Gassensor" kann beispielsweise ein galvanischer, paramagnetischer oder optischer Sensor verstanden werden. Ein solcher Gassensor kann in einem Hauptstrom und/oder einem Nebenstrom der ausgeatmeten Atemluft angeordnet sein und/oder als Druck- und/oder Flusssensor ausgebildet sein.

Weitere Aspekte der Erfindung betreffen ein Computerprogramm und ein computerlesbares Medium, auf dem das Computerprogramm gespeichert ist.

Das Computerprogramm umfasst Befehle, die einen Prozessor (beispielsweise den Prozessor der vor- und nachstehend beschriebenen Datenverarbeitungsvorrichtung) beim Ausführen des Computerprogramms durch den Prozessor veranlassen, mindestens eines der vor- und nachstehend beschriebenen Verfahren auszuführen.

Das computerlesbare Medium kann ein flüchtiger oder nicht flüchtiger Datenspeicher sein. Beispielsweise kann das computerlesbare Medium eine Festplatte, ein USB-Speichergerät *(universal serial bus),* ein RAM *(random-access memory),* ein ROM *(read-only memory),* ein EPROM *(erasable programmable read-only memory),* ein EEPROM *(electrically erasable programmable read-only memory),* ein Flash-Speicher oder eine Kombination von mindestens zwei dieser Beispiele sein. Das computerlesbare Medium kann auch ein Datenkommunikationsnetzwerk, das das Herunterladen von Programmcode ermöglicht (z. B. über das Internet), oder eine Cloud sein.

Es wird darauf hingewiesen, dass Merkmale der vor- und nachstehend beschriebenen Verfahren auch Merkmale des Computerprogramms und/oder des computerlesbaren Mediums sein können (und umgekehrt).

Im Folgenden werden verschiedene Ausführungsformen der Erfindung beschrieben. Diese Ausführungsformen sind nicht als Beschränkung des Umfangs der Erfindung zu verstehen.

Gemäß einer Ausführungsform können die Messdaten den volumenabhängigen Verlauf bezogen auf einen einzelnen Atemzug des Patienten anzeigen. Anders ausgedrückt kann jedem Punkt des volumenabhängigen Verlaufs ein bestimmter Anteil eines bei einem einzelnen Atemzug vom Patienten ausgeatmeten Gesamtvolumens einer das Atemgas umfassenden Atemluft zugeordnet sein. Dementsprechend kann dem Anfang des volumenabhängigen Verlaufs ein Volumen von null und dem Ende des volumenabhängigen Verlaufs ein Volumen gleich dem Gesamtvolumen zugeordnet sein. Ein solches Gesamtvolumen kann auch als Atemzug- oder Tidalvolumen bezeichnet werden. Es ist möglich, dass die Messdaten während des einzelnen Atemzugs erzeugt wurden und/oder bei jedem Atemzug neu erzeugt und/oder neu empfangen werden.

Mit anderen Worten können die Messdaten eine Reihe von Konzentrationswerten für die Konzentration des Atemgases im Atemluftstrom und eine Reihe von Volumenwerten für das Atemluftvolumen umfassen. Jeder Volumenwert kann ein Wert aus einem durch einen unteren Grenzwert und einen oberen Grenzwert begrenzten Wertebereich sein, wobei der untere Grenzwert null ist und der obere Grenzwert ein bei einem einzelnen Atemzug vom Patienten ausgeatmetes Gesamtvolumen anzeigt und jedem Konzentrationswert ein anderer Volumenwert zugeordnet ist.

Gemäß einer Ausführungsform können die Messdaten ferner einen dem volumenabhängigen Verlauf zugeordneten positiven endexspiratorischen Druck zum Beatmen des Patienten anzeigen. In diesem Fall können die Eingabedaten den positiven endexspiratorischen Druck umfassen und/oder unter Berücksichtigung des positiven endexspiratorischen Drucks erzeugt werden. Dies ermöglicht eine genauere Schätzung im Vergleich zu einer Ausführungsform ohne Berücksichtigung des positiven endexspiratorischen Drucks.

Gemäß einer Ausführungsform kann eine mathematische Funktion, die zumindest einen Abschnitt des volumenabhängigen Verlaufs näherungsweise definiert, unter Verwendung der Messdaten bestimmt werden. Dabei kann mindestens einer der Werte in der Matrix unter Verwendung der mathematischen Funktion berechnet werden. Mithilfe der mathematischen Funktion können geeignete Parameter auf vorhersehbare und transparente Weise bestimmt werden. Die mathematische Funktion kann eine einzelne mathematische Funktion oder eine Kombination mehrerer einzelner mathematischer Funktionen sein.

Gemäß einer Ausführungsform können die Eingabedaten eine Matrix von Werten für 2 bis 20, 10 bis 20 oder 10 bis 15 verschiedene Parameter bezüglich des volumenabhängigen Verlaufs umfassen. Auf diese Weise kann der Verbrauch von Rechenressourcen im Vergleich zu einer Ausführungsform mit größeren Eingabematrizen deutlich verringert werden.

Unter "Parameter" kann vor- und nachstehend ein für eine Beatmung eines Patienten relevanter Beatmungsparameter verstanden werden. Dabei kann jeder Wert in der Matrix einem der verschiedenen Parameter zugeordnet sein. Dementsprechend kann die Matrix je nach Anzahl der verschiedenen Parameter beispielsweise 2 bis 20, 10 bis 20 oder 10 bis 15 Eingabewerte umfassen. Die Matrix kann als ein ein-, zwei- oder dreidimensionaler Vektor aufgefasst werden. Beispielsweise kann das Machine-Learning-Modul konfiguriert sein, um diese Eingabewerte in einen einzigen Ausgabewert, der die Konzentration des Atemgases im Blut des Patienten anzeigt, umzuwandeln.

Gemäß einer Ausführungsform können die Messdaten in mehreren aufeinanderfolgenden Zeitschritten erzeugt worden sein. In diesem Fall kann die mathematische Funktion unter Verwendung der Messdaten aus verschiedenen Zeitschritten bestimmt werden, beispielsweise durch Regression. Ein einzelner Zeitschritt kann beispielsweise so lange wie ein einzelner Atemzug des Patienten dauern. Die jeweilige Dauer der Zeitschritte kann aber auch fest vorgegeben sein und/oder beispielsweise in einer Größenordnung von 1 ms, 10 ms, 100 ms, 1 s oder 10 s liegen.

Gemäß einer Ausführungsform kann die mathematische Funktion gemäß dem Levenberg-Marquardt-Algorithmus bestimmt werden. Unter "Levenberg-Marquardt-Algorithmus" kann ein spezieller numerischer Optimierungsalgorithmus zur Lösung nicht linearer Ausgleichsprobleme mithilfe der Methode der kleinsten Quadrate verstanden werden. Der Algorithmus kann als eine Kombination des Gauß-Newton-Verfahrens mit einer Regularisierungstechnik, die absteigende Funktionswerte erzwingt, aufgefasst werden. Dies ermöglicht eine genauere und recheneffizientere Approximation als eine Implementierung der klassischen Fowler'schen Methode, und zwar auch bei stärkeren Schwankungen des volumenabhängigen Verlaufs zwischen aufeinanderfolgenden Atemzügen und/oder zwischen verschiedenen Patienten.

Gemäß einer Ausführungsform können die verschiedenen Parameter mindestens einen der folgenden Parameter umfassen: ein bei einem einzelnen Atemzug vom Patienten ausgeatmetes Gesamtvolumen des Atemgases; ein bei einem einzelnen Atemzug vom Patienten ausgeatmetes Gesamtvolumen einer das Atemgas umfassenden Atemluft (auch Atemzug- oder Tidalvolumen genannt); ein Atemminutenvolumen; eine alveoläre Ventilation; einen Atemwegstotraum; einen gemischt exspiratorischen Partialdruck des Atemgases; einen endtidalen Partialdruck des Atemgases; einen positiven endexspiratorischen Druck zum Beatmen des Patienten.

Das Atemminutenvolumen kann als das Produkt des Atemzugvolumens und der Atemfrequenz aufgefasst werden.

Die alveoläre Ventilation (*V̇_{A}*) kann als das Produkt der Atemfrequenz und der Differenz zwischen dem Atemzugvolumen und dem (anatomischen) Totraum aufgefasst werden.

Unter "Atemwegstotraum" kann allgemein ein Anteil des Atemzugvolumens verstanden werden, der bei jedem Atemzug in den luftleitenden Atemwegen verbleibt und somit das alveoläre Kompartiment nicht erreicht.

Der endtidale Partialdruck des Atemgases kann als die Konzentration des Atemgases in dem vom Patienten ausgeatmeten Atemluftstrom am Ende eines einzelnen Atemzugs aufgefasst werden.

Der gemischt exspiratorische Partialdruck des Atemgases kann als ein bestimmter Bruchteil des endtidalen Partialdrucks aufgefasst werden und/oder beispielsweise einer mittleren Konzentration des Atemgases in dem vom Patienten ausgeatmeten Atemluftstrom bei einem einzelnen Atemzug entsprechen.

Gemäß einer Ausführungsform kann der volumenabhängige Verlauf in mindestens drei aufeinanderfolgende charakteristische Ausatmungsphasen bei einem einzelnen Atemzug des Patienten unterteilt werden. In diesem Fall können die verschiedenen Parameter mindestens einen der folgenden Parameter umfassen: ein erstes Atemgasvolumen als ein in einer ersten (beispielsweise frühesten) der Ausatmungsphasen vom Patienten ausgeatmetes Volumen des Atemgases; ein zweites Atemgasvolumen als ein in einer zweiten (beispielsweise mittleren) der Ausatmungsphasen vom Patienten ausgeatmetes Volumen des Atemgases; ein drittes Atemgasvolumen als ein in einer dritten (beispielsweise letzten oder vorletzten) der Ausatmungsphasen vom Patienten ausgeatmetes Volumen des Atemgases; eine (beispielsweise durchschnittliche) Steigung des volumenabhängigen Verlaufs in mindestens einer der Ausatmungsphasen, insbesondere in einer letzten (oder vorletzten) der Ausatmungsphasen.

Die mittlere Ausatmungsphase kann als eine zeitlich zwischen der frühesten und der letzten (oder vorletzten) Ausatmungsphase liegende Ausatmungsphase aufgefasst werden. Es ist möglich, dass die erste Ausatmungsphase unmittelbar in die zweite Ausatmungsphase und/oder die zweite Ausatmungsphase unmittelbar in die dritte Ausatmungsphase übergeht.

Unter "Steigung" kann eine (beispielsweise durchschnittliche) Änderungsrate der Konzentration des Atemgases im Atemgasstrom bezogen auf die jeweilige(n) Ausatmungsphase(n) verstanden werden. Die Steigung kann zusätzlich in geeigneter Weise normiert werden, um eine normierte Steigung zu erhalten, die dann als einer der Parameter verwendet werden kann.

Die Ausatmungsphasen können beispielsweise mithilfe der vorgenannten mathematischen Funktion und/oder entsprechend der Fowler'schen Methode bestimmt worden sein. Bei den Ausatmungsphasen kann es sich um die typischen (drei oder vier) Phasen eines volumetrischen Kapno- oder Oxigramms handeln. Die Ausatmungsphasen können sich signifikant in ihrer Länge und/oder in der (beispielsweise durchschnittlichen) Steigung des volumenabhängigen Verlaufs voneinander unterscheiden.

Beispielsweise kann im Fall eines Kapnogramms die erste Ausatmungsphase vom Beginn der Ausatmung bis zu einem ersten Punkt reichen, an dem die Änderungsrate der zweiten Ableitung des volumenabhängigen Verlaufs ihr Maximum erreicht bzw. die dritte Ableitung des volumenabhängigen Verlaufs ihr linksseitiges Maximum erreicht. Die zweite Ausatmungsphase kann vom ersten Punkt bis zu einem zweiten Punkt reichen, an dem die dritte Ableitung des volumenabhängigen Verlaufs ihr rechtsseitiges Maximum erreicht. Die dritte Ausatmungsphase kann vom zweiten Punkt bis zum Ende der Ausatmung reichen. Unter dem Begriff "volumenabhängiger Verlauf" kann hier auch eine Approximation, beispielsweise in Form der vorgenannten mathematischen Funktion, verstanden werden.

Bei der ersten Ausatmungsphase kann es sich um die früheste Phase der Ausatmung (10 % bis 12 % des gesamten Atemzugs) handeln, in der kaum oder kein Kohlendioxid in der Atemluft enthalten ist. Bei der zweiten Ausatmungsphase kann es sich um eine Phase des größten (durchschnittlichen) Anstiegs der Kohlendioxidkonzentration im Atemluftstrom handeln (15 % bis 18 % des gesamten Atemzugs). Bei der dritten Ausatmungsphase kann es sich um eine Phase handeln, in der die Kohlendioxidkonzentration im Atemluftstrom - im Gegensatz zu den vorangehenden Ausatmungsphasen - überwiegend durch die aus den Alveolen kommenden Gase bestimmt wird (70 % bis 75 % des gesamten Atemzugs).

Gemäß einer Ausführungsform kann mindestens ein normiertes Atemgasvolumen durch Dividieren eines der drei Atemgasvolumen durch ein bei dem einzelnen Atemzug vom Patienten ausgeamtetes Gesamtvolumen (des Atemgases oder einer das Atemgas umfassenden Atemluft) bestimmt werden. In diesem Fall können die Parameter das mindestens eine normierte Atemgasvolumen zusätzlich oder alternativ zum jeweiligen (nicht normierten) ersten, zweiten bzw. dritten Atemgasvolumen umfassen.

Gemäß einer Ausführungsform kann das Machine-Learning-Modul ein künstliches neuronales Netz umfassen. Dementsprechend kann es sich bei den Gewichten des Machine-Learning-Moduls um Gewichte des künstlichen neuronalen Netzes handeln. Das künstliche neuronale Netz kann eine Eingabeschicht zum Eingeben der Eingabedaten, eine Ausgabeschicht zum Ausgeben der Ausgabedaten und mindestens eine trainierbare Zwischenschicht zwischen der Eingabeschicht und der Ausgabeschicht umfassen. Das künstliche neuronale Netz kann beispielsweise mindestens einen der folgenden Netztypen umfassen: ein mehrlagiges Perzeptron, ein Deep Neural Network (DNN), ein Convolutional Neural Network (CNN), ein Recurrent Neural Network (RNN), ein Long Short-Term Memory (LSTM). Beispielsweise kann das künstliche neuronale Netz höchstens 30, höchstens 15 oder höchstens 5 trainierbare Zwischenschichten umfassen. Eine derartige Netzarchitektur ist besonders recheneffizient und ermöglicht dennoch eine ausreichend genaue Schätzung.

Gemäß einer Ausführungsform kann das künstliche neuronale Netz als ein adaptives Neuro-Fuzzy-Inferenzsystem (ANFIS) implementiert sein. Ein solches Inferenzsystem kann auf einem Takagi-Sugeno-Regler und/oder Tsukamoto-Regler basieren und/oder eine Reihe von Fuzzy-If-Then-Regeln umfassen, die trainiert werden können, um nicht lineare Funktionen zu approximieren. Die Architektur des ANFIS kann beispielsweise fünf Schichten umfassen, darunter eine sogenannte Fuzzification-Schicht als einer ersten der fünf Schichten. Diese Ausführungsform ermöglicht es, die Vorteile eines künstlichen neuronalen Netzes mit den Vorteilen einer Fuzzylogik in einem einzigen Framework zu vereinen.

Gemäß einer Ausführungsform können die Messdaten mindestens erste Messdaten und zweite Messdaten umfassen. In diesem Fall können die ersten Messdaten einen volumenabhängigen Verlauf einer Konzentration eines ersten Atemgases im Atemluftstrom abhängig vom Atemluftvolumen anzeigen und die zweiten Messdaten einen volumenabhängigen Verlauf einer Konzentration eines vom ersten Atemgas abweichenden zweiten Atemgases im Atemluftstrom abhängig vom Atemluftvolumen anzeigen. Dementsprechend können die Ausgabedaten eine Konzentration des ersten Atemgases und des zweiten Atemgases im Blut des Patienten anzeigen. Diese Ausführungsform ermöglicht die gleichzeitige Schätzung der Konzentrationen verschiedener Atemgase im Blut des Patienten, beispielsweise von Kohlendioxid und Sauerstoff.

Gemäß einer Ausführungsform können die Eingabedaten aus den ersten Messdaten erzeugte erste Eingabedaten und aus den zweiten Messdaten erzeugte zweite Eingabedaten umfassen. Dabei können die ersten Eingabedaten in ein erstes künstliches neuronales Netz eingegeben werden und es können erste Ausgabedaten, die die Konzentration des ersten Atemgases im Blut des Patienten anzeigen, durch das erste künstliche neuronale Netz ausgegeben werden. Analog dazu können die zweiten Eingabedaten in ein zweites künstliches neuronales Netz eingegeben werden und es können zweite Ausgabedaten, die die Konzentration des zweiten Atemgases im Blut des Patienten anzeigen, durch das zweite künstliche neuronale Netz ausgegeben werden. Dementsprechend können die Ausgabedaten die ersten Ausgabedaten und die zweiten Ausgabedaten umfassen. Bei den Gewichten des Machine-Learning-Moduls kann es sich beispielsweise um Gewichte des ersten bzw. zweiten künstlichen neuronalen Netzes handeln. Die zwei künstlichen neuronalen Netze können in ihrer Architektur und/oder in ihren Gewichten voneinander abweichen oder miteinander übereinstimmen und/oder getrennt voneinander trainiert worden sein. Beispielsweise kann mindestes eines oder jedes der zwei künstlichen neuronalen Netze in seiner Architektur mit dem vorstehend beschriebenen Netz übereinstimmen und/oder als ein adaptives Neuro-Fuzzy-Inferenzsystem implementiert sein. Alternativ können die ersten und die zweiten Eingabedaten in ein gemeinsames künstliches neuronales Netz, beispielsweise in das vorstehend beschriebene Netz, eingegeben werden.

Beispielsweise können die ersten Eingabedaten eine Matrix erster Werte für verschiedene erste Parameter bezüglich des volumenabhängigen Verlaufs der ersten Messdaten umfassen und/oder die zweiten Eingabedaten eine Matrix zweiter Werte für verschiedene zweite Parameter bezüglich des volumenabhängigen Verlaufs der zweiten Messdaten umfassen. Die ersten Parameter können in ihrer Anzahl und/oder ihrem Typ mit den zweiten Parametern übereinstimmen und/oder von den zweiten Parametern abweichen. Es ist möglich, dass mindestens einer der ersten Werte unter Verwendung einer ersten mathematischen Funktion, die zumindest einen Abschnitt des volumenabhängigen Verlaufs der ersten Messdaten (beispielsweise aus verschiedenen Zeitschritten) näherungsweise definiert, bestimmt wird und/oder mindestens einer der zweiten Werte unter Verwendung einer zweiten mathematischen Funktion, die zumindest einen Abschnitt des volumenabhängigen Verlaufs der zweiten Messdaten (beispielsweise aus verschiedenen Zeitschritten) näherungsweise definiert, bestimmt wird.

### Kurze Beschreibung der Zeichnungen

Im Folgenden werden Ausführungsformen der Erfindung mit Bezug auf die beigefügten Zeichnungen beschrieben. Weder die Beschreibung noch die Zeichnungen sind als Beschränkung des Umfangs der Erfindung zu verstehen.
Fig. 1 zeigt ein Beatmungsgerät gemäß einer Ausführungsform der Erfindung.
Fig. 2 zeigt ein Flussdiagramm zur Veranschaulichung einer Ausführungsform eines Verfahrens zum Schätzen einer Konzentration eines Atemgases im Blut eines Patienten.
Fig. 3 zeigt ein Flussdiagramm zur Veranschaulichung einer Ausführungsform eines Verfahrens zum Trainieren eines Machine-Learning-Moduls für ein Beatmungsgerät.
Fig. 4 zeigt ein Kapnogramm zur Verwendung in einem Verfahren gemäß einer Ausführungsform der Erfindung.
Fig. 5 zeigt ein Machine-Learning-Modul zur Verwendung in einem Verfahren gemäß einer Ausführungsform der Erfindung.
Fig. 6 zeigt ein Flussdiagramm zur Veranschaulichung der Erzeugung von Trainingsdatensätzen in einem Verfahren gemäß einer Ausführungsform der Erfindung.

Die Figuren sind rein schematisch und nicht maßstabsgetreu. Werden in verschiedenen Zeichnungen gleiche Bezugszeichen verwendet, so bezeichnen diese Bezugszeichen gleiche oder gleichwirkende Merkmale.

### Ausführungsformen der Erfindung

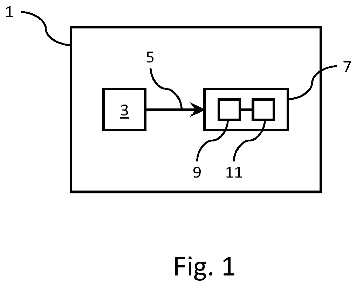

Fig. 1 zeigt ein Beatmungsgerät 1 zum invasiven und/oder nicht invasiven Beatmen eines Patienten. Das Beatmungsgerät 1 umfasst eine Sensorik 3 zum Erzeugen von Messdaten 5 und eine Datenverarbeitungsvorrichtung 7 mit einem Speicher 9 und einem Prozessor 11, der konfiguriert ist, um ein im Speicher 9 gespeichertes Computerprogramm zum Verarbeiten der Messdaten 5 in mindestens einem der nachstehend beschriebenen Verfahren auszuführen.

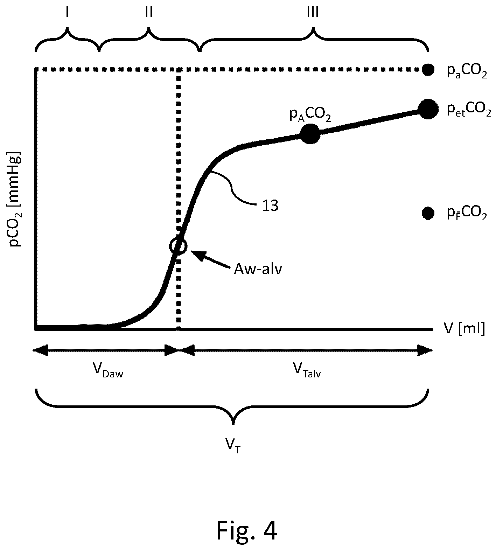

Die Messdaten 5 definieren einen volumenabhängigen Verlauf 13 (siehe Fig. 4) einer Konzentration eines Atemgases (z. B. pCO₂) in einem vom Patienten ausgeatmeten Atemluftstrom abhängig von einem vom Patienten ausgeatmeten Atemluftvolumen.

Insbesondere können die Messdaten 5 den volumenabhängigen Verlauf 13 bezogen auf einen einzelnen Atemzug des Patienten anzeigen. Zudem ist es möglich, dass die Messdaten 5 einen dem volumenabhängigen Verlauf 13 zugeordneten positiven endexspiratorischen Druck (kurz PEEP) zum Beatmen des Patienten anzeigen. Beispielsweise können die Messdaten 5 - wie in Fig. 4 gezeigt - ein durch volumetrische Kapnographie nicht invasiv erzeugtes Kapnogramm codieren, das den volumenabhängigen Verlauf 13 der Konzentration von Kohlendioxid als dem Atemgas anzeigt. Zusätzlich oder alternativ können die Messdaten 5 ein durch volumetrische Oxigraphie nicht invasiv erzeugtes Oxigramm codieren, das den volumenabhängigen Verlauf 13 der Konzentration von Sauerstoff als dem Atemgas anzeigt.

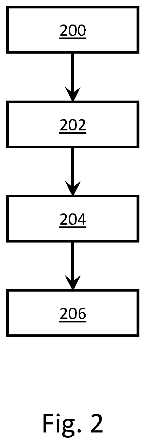

Fig. 2 zeigt ein Beispiel für ein Verfahren zum Schätzen der Konzentration des Atemgases im Blut des Patienten. Das Verfahren wird von der Datenverarbeitungsvorrichtung 7 ausgeführt und umfasst die folgenden Schritte.

In einem Schritt 200 werden die Messdaten 5 empfangen.

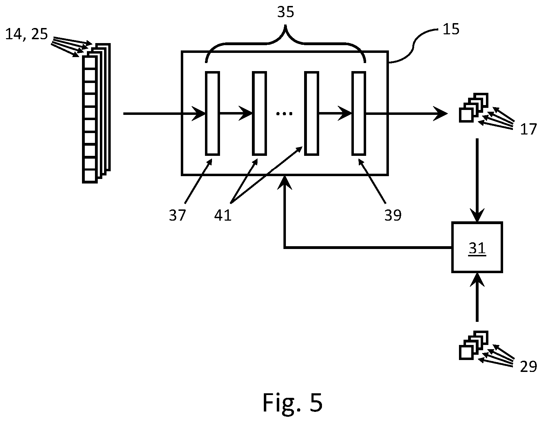

In einem Schritt 202 werden Eingabedaten 14 aus den Messdaten 5 erzeugt (siehe auch Fig. 5). Die Eingabedaten 14 umfassen eine Matrix von Werten für verschiedene Parameter bezüglich des volumenabhängigen Verlaufs 13.

In einem Schritt 204 werden die Eingabedaten 14 in ein Machine-Learning-Modul 15 eingegeben, das trainiert wurde, um die Eingabedaten 14 in Ausgabedaten 17 umzuwandeln, die die (geschätzte) Konzentration des Atemgases im Blut des Patienten anzeigen (unter "Modul" kann vor- und nachstehend ein Soft- und/oder Hardwaremodul verstanden werden). Beispielsweise können die Ausgabedaten 17 einen Schätzwert für den arteriellen Kohlendioxidpartialdruck (pₐCO₂) und/oder einen Schätzwert für den arteriellen Sauerstoffpartialdruck (pₐO₂) umfassen.

In einem Schritt 206 werden die Ausgabedaten 17 durch das Machine-Learning-Modul 15 ausgegeben.

Anschließend können die Ausgabedaten 17 zusätzlich weiterverarbeitet werden, beispielsweise um die Ausgabedaten 17 in geeigneter Weise auf einem Display des Beatmungsgeräts 1 anzuzeigen und/oder über eine drahtlose und/oder drahtgebundene Datenkommunikationsverbindung an ein externes Gerät wie z. B. einen Server, einen PC, einen Laptop, ein Tablet, ein Smartphone oder eine Smartwatch zu senden.

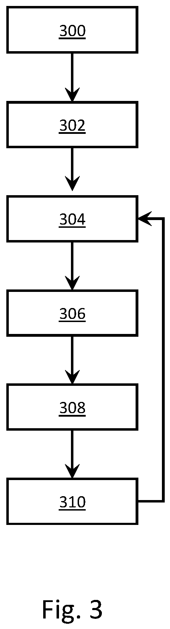

Fig. 3 zeigt ein Beispiel für ein Verfahren zum Trainieren des Machine-Learning-Moduls 15. Es ist möglich, dass das Verfahren ebenfalls von der Datenverarbeitungsvorrichtung 7 ausgeführt wird. Zusätzlich oder alternativ kann das Verfahren von einer anderen Datenverarbeitungsvorrichtung außerhalb des Beatmungsgeräts 1 ausgeführt werden. Das Verfahren umfasst die folgenden Schritte.

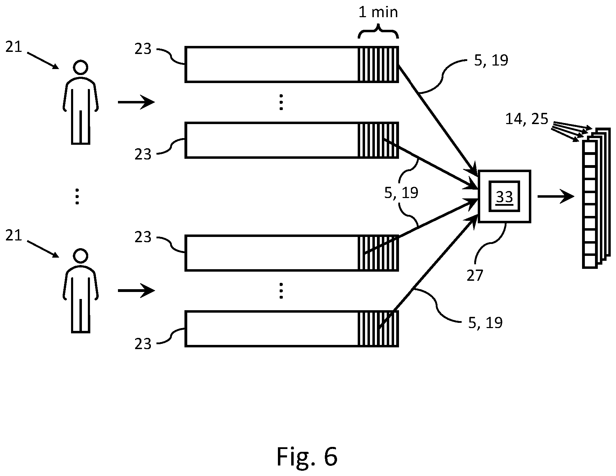

In einem Schritt 300 werden mehrere Messdatensätze 19, die jeweils die Messdaten 5 umfassen, empfangen (siehe auch Fig. 6). Die Messdaten 5 der verschiedenen Messdatensätze 19 sind zumindest teilweise verschiedenen Patienten 21 zugeordnet. Beispielsweise können die Messdatensätze 19 beim maschinellen Beatmen der

Patienten 21 in mehreren kontrollierten Beatmungsschritten 23 einer bestimmten Länge von z. B. 2 min, 5 min oder 10 min erzeugt worden sein. Dabei kann jeder Messdatensatz 19 die Messdaten 5 für genau einen Atemzug des jeweiligen Patienten 21 umfassen.

In einem Schritt 302 werden aus den Messdatensätzen 19 der verschiedenen Patienten 21 mehrere Trainingsdatensätze 25 erzeugt, wobei jeder Trainingsdatensatz 25 einem der Patienten 21 zugeordnet ist und eine Matrix von Werten für verschiedene Parameter bezüglich des jeweiligen volumenabhängigen Verlaufs 13 umfasst. Die Trainingsdatensätze 25 können beispielsweise durch ein geeignet konfiguriertes Auswahlmodul 27, das als ein weiteres Machine-Learning-Modul implementiert sein kann, erzeugt werden. Es ist möglich, dass zum Erzeugen der Trainingsdatensätze 25 nur Messdatensätze 19 verwendet werden, die jeweils in einem bestimmten Zeitabschnitt der Beatmungsschritte 23 erzeugt wurden, beispielsweise in der letzten Minute, wie in Fig. 6 gezeigt. Bei einer kontrollierten Atemzugdauer von 2 s resultieren somit 30 Messdatensätze pro Beatmungsschritt 23 eines jeden Patienten 21. Aus jedem dieser Messdatensätze 19 kann dann beispielsweise ein Trainingsdatensatz 25 erzeugt werden.

In einem Schritt 304 wird jeder Trainingsdatensatz 25 als Eingabedaten 14 in das Machine-Learning-Modul 15 eingegeben. Das Machine-Learning-Modul 15 wandelt die Eingabedaten 14 in Ausgabedaten 17 um, die die (geschätzte) Konzentration des Atemgases im Blut des jeweiligen Patienten 21 anzeigen.

In einem Schritt 306 werden die Ausgabedaten 17 durch das Machine-Learning-Modul 15 ausgegeben.

In einem Schritt 308 wird eine Abweichung der Ausgabedaten 17 von Zieldaten 29, die dem jeweiligen Trainingsdatensatz 25 bzw. den jeweiligen Eingabedaten 14 zugeordnet sind, bestimmt.

In einem Schritt 310 werden schließlich die Gewichte des Machine-Learning-Moduls 15 in einem Optimierungsverfahren angepasst, um die Abweichung zu verringern, beispielsweise in einem Gradientenverfahren mit Backpropagation.

Die Schritte 308, 310 können beispielsweise durch ein geeignet konfiguriertes Optimierungsmodul 31 ausgeführt werden.

Zweckmäßigerweise wird das Machine-Learning-Modul 15 in mehreren aufeinanderfolgenden Zeitschritten trainiert, bis die Abweichung einen akzeptablen Wert erreicht. Dabei können beispielsweise in jedem Zeitschritt die Schritte 304 bis 310 ausgeführt werden.

Wie in Fig. 5 und Fig. 6 gezeigt, kann die Matrix ein eindimensionaler Vektor sein und/oder Werte für mindestens 2, mindestens 5, mindestens 10 verschiedene Parameter, insbesondere für 11 oder 12 verschiedene Parameter, umfassen. Die Anzahl der Parameter sollte nicht zu groß sein, beispielsweise nicht größer als 15, 20 oder 30, um den Verbrauch von Rechenressourcen niedrig zu halten. Die Anzahl der Werte in der Matrix kann mit der Anzahl der verschiedenen Parameter übereinstimmen, d. h., jeder Wert in der Matrix kann genau einen der verschiedenen Parameter definieren.

Die verschiedenen Parameter können je nach Ausführungsform mindestens einen der folgenden Parameter in Bezug auf den jeweiligen Patienten umfassen (siehe auch Fig. 4): ein bei einem einzelnen Atemzug vom Patienten ausgeatmetes Gesamtvolumen des Atemgases; ein bei einem einzelnen Atemzug vom Patienten ausgeatmetes Gesamtvolumen einer das Atemgas umfassenden Atemluft, auch Atemzug- oder Tidalvolumen genannt (V_{T}); ein Atemminutenvolumen; eine alveoläre Ventilation; einen Atemwegstotraum (V_{Daw}); einen gemischt exspiratorischen Partialdruck des Atemgases (z. B. p_{E̅}CO₂); einen endtidalen Partialdruck des Atemgases (z. B. pₑₜCO₂); einen positiven endexspiratorischen Druck zum Beatmen des Patienten.

Zudem kann der volumenabhängige Verlauf 13 der jeweiligen Messdaten 5 in mindestens drei aufeinanderfolgende charakteristische Ausatmungsphasen bei einem einzelnen Atemzug des jeweiligen Patienten unterteilt werden. In diesem Fall können die verschiedenen Parameter mindestens einen der folgenden Parameter umfassen: ein erstes Atemgasvolumen als ein in einer ersten Ausatmungsphase I vom Patienten ausgeatmetes Volumen des Atemgases; ein zweites Atemgasvolumen als ein in einer zweiten Ausatmungsphase II vom Patienten ausgeatmetes Volumen des Atemgases; ein drittes Atemgasvolumen als ein in einer dritten Ausatmungsphase III vom Patienten ausgeatmetes Volumen des Atemgases; eine durchschnittliche Steigung des volumenabhängigen Verlaufs 13 in mindestens einer der Ausatmungsphasen I, II, III, insbesondere in der dritten Ausatmungsphase III.

Optional können die drei Atemgasvolumen jeweils durch Dividieren durch das Atemzugvolumen normiert werden. In diesem Fall können die verschiedenen Parameter das jeweilige normierte Atemgasvolumen umfassen.

Die Werte für diese Parameter können zumindest teilweise unter Verwendung einer speziellen mathematischen Funktion 33 aus den Messdaten 5 berechnet werden. Die mathematische Funktion 33 kann auch dazu verwendet werden, die drei Ausatmungsphasen I, II, III zu ermitteln. Alternativ können die verschiedenen Parameter zumindest teilweise unter Verwendung eines zusätzlichen Machine-Learning-Moduls bestimmt werden.

Bei der mathematischen Funktion 33 kann es sich um eine Approximation zumindest eines Abschnitts des volumenabhängigen Verlaufs 13 handeln. Die Approximation kann beispielsweise durch eine Regressionsanalyse von Messdaten 5, die in mehreren aufeinanderfolgenden Zeitschritten, z. B. bei mehreren Atemzügen, in Bezug auf einen Patienten erzeugt wurden, bestimmt werden. Eine besonders genaue und recheneffiziente Approximation kann erreicht werden, wenn die mathematische Funktion 33 gemäß dem Levenberg-Marquardt-Algorithmus bestimmt wird.

Das Machine-Learning-Modul 15 kann beispielsweise als ein künstliches neuronales Netz 35 (siehe Fig. 5) mit einer Eingabeschicht 37 zum Eingeben der Eingabedaten 14, einer Ausgabeschicht 39 zum Ausgeben der Ausgabedaten 17 und mindestens einer trainierbaren Zwischenschicht 41 zum Umwandeln der Eingabedaten 14 in die Ausgabedaten 17 implementiert sein. Alternativ kann das Machine-Learning-Modul 15 als eine Kombination mehrerer künstlicher neuronaler Netze implementiert sein. Möglich sind auch andere Machine-Learning-Algorithmen wie z. B. ein Entscheidungsbaum, ein Random Forest, ein k-nächste-Nachbarn-Algorithmus, eine Support-Vector-Maschine, ein Bayes-Klassifikator, ein k-Means-Algorithmus, ein genetischer Algorithmus, ein Kernelregressionsalgorithmus oder ein Diskriminanzanalyse-Algorithmus.

Um ein schnelles Training - beispielsweise auf dem Beatmungsgerät 1 - und die Verwendung kostengünstiger Hardware zu ermöglichen, sollte das Netz 35 nicht übermäßig komplex sein. Eine Architektur mit höchstens 10 trainierbaren Zwischenschichten 41 erwies sich in Versuchen als ein guter Kompromiss zwischen Effizienz und Genauigkeit. Besonders günstig ist ein Netz 35 in Form eines adaptiven Neuro-Fuzzy-Inferenzsystems (ANFIS). Möglich sind aber auch deutlich komplexere DNN-Architekturen.

Das Machine-Learning-Modul 15 kann auch trainiert werden, um gleichzeitig die Konzentration verschiedener Atemgase im Blut des Patienten zu schätzen.

In diesem Fall können die Messdaten 5 beispielsweise erste Messdaten und zweite Messdaten umfassen, wobei die ersten Messdaten einen volumenabhängigen Verlauf 13 einer Konzentration eines ersten Atemgases, beispielsweise Kohlendioxid, im Atemluftstrom abhängig vom Atemluftvolumen anzeigen und die zweiten Messdaten einen volumenabhängigen Verlauf einer Konzentration eines zweiten Atemgases, beispielsweise Sauerstoff, im Atemluftstrom abhängig vom Atemluftvolumen anzeigen. Dementsprechend können die Trainingsdatensätze 25 bzw. die Eingabedaten 14 aus den ersten und den zweiten Messdaten erzeugt werden und die Ausgabedaten 17 können eine (geschätzte) Konzentration des ersten Atemgases und des zweiten Atemgases im Blut des jeweiligen Patienten anzeigen.

Hierzu können die Eingabedaten 14 entweder in ein gemeinsames künstliches neuronales Netz 35 oder in zwei separat trainierbare bzw. trainierte künstliche neuronale Netze eingegeben werden. Im zweiten Fall können beispielsweise aus den ersten Messdaten erste Eingabedaten für ein erstes Netz und zweite Eingabedaten aus den zweiten Messdaten für ein zweites Netz erzeugt werden. Dementsprechend werden erste Ausgabedaten, die die Konzentration des ersten Atemgases im Blut des Patienten anzeigen, durch das erste Netz und zweite Ausgabedaten, die die Konzentration des zweiten Atemgases im Blut des Patienten anzeigen, durch das zweite Netz ausgegeben.

Das Training des Machine-Learning-Moduls 15 kann beispielsweise folgende Schritte umfassen.

Zunächst werden Blutproben von verschiedenen Patienten genommen und es wird für jede Blutprobenentnahme ein Entnahmezeitpunkt notiert.

Die Blutproben werden analysiert, um einen tatsächlichen arteriellen Kohlendioxid- und/oder Sauerstoffpartialdruck des jeweiligen Patienten zu bestimmen.

Gleichzeitig mit und/oder kurz vor und/oder kurz nach der Blutprobenentnahme wird der Kohlendioxid- bzw. Sauerstoffpartialdruck in dem vom jeweiligen Patienten ausgeatmeten Atemluftstrom bei mehreren Atemzügen zumindest während der Ausatmung sensorisch erfasst. Zudem wird der Fluss des Atemluftstroms zumindest während der Ausatmung sensorisch erfasst.

Durch Integration des Flusses wird das vom Patienten ausgeatmete Atemluftvolumen für jeden Atemzug bestimmt.

Hierauf werden aus den zeitbasierten Verläufen des Kohlendioxid- bzw. Sauerstoffpartialdrucks im Atemluftstrom und des Atemluftvolumens entsprechende volumetrische Kapno- bzw. Oxigramme erzeugt.

Anschließend wird in einem Optimierungsverfahren, beispielsweise mithilfe einer Regressionsanalyse, eine mathematische Funktion bestimmt, die die Kurven in den Kapno- bzw. Oxigrammen näherungsweise definiert.

Anhand der mathematischen Funktion werden dann verschiedene Parameter bestimmt, die bestimmte Merkmale der Kurven beschreiben.

Diese Parameter werden zusammen mit den jeweiligen Werten des tatsächlichen arteriellen Kohlendioxid- bzw. Sauerstoffpartialdrucks verwendet, um das Machine-Learning-Modul 15 zu trainieren.

Die Praxistauglichkeit des Verfahrens konnte unter anderem im folgenden Versuch bestätigt werden.

Bei 14 lungengespülten Versuchstieren wurde der arterielle Kohlendioxidpartialdruck (pₐCO₂) kontinuierlich mittels eines optischen intravaskulären Katheters erfasst. Gleichzeitig wurde ein Kapnogramm bei jedem Atemzug erfasst. Die Tiere wurden mechanisch mit fixen Einstellungen beatmet, wobei der positive endexspiratorische Druck in mehreren Stufen von 0 bis 22 cmH₂O variiert wurde. Die resultierenden 8599 Datenpunkte - jeweils ein pₐCO₂-Wert gepaart mit einem Satz von 12 Parametern, die aus dem Kapnogramm eines Atemzugs abgeleitet wurden - wurden in ein ANFIS-Modell eingegeben. Die Datenpunkte wurden in einen ersten Satz von 7370 Datenpunkten (85 %) zum Trainieren des Modells und einen zweiten Satz von 1229 Datenpunkten (15 %) zum Testen des trainierten Modells aufgeteilt. Die ANFIS-Analyse wurde in 10 unabhängigen Schritten wiederholt, wobei die einzugebenden Datenpunkte jeweils nach dem Zufallsprinzip ausgewählt wurden.

Das Bland-Altman-Diagramm für die 10 unabhängig voneinander getesteten ANFIS-Modelle ergab eine mittlere Abweichung von 0,03 ± 0,03 mmHg zwischen geschätztem und tatsächlichem pₐCO₂-Wert bei einem Übereinstimmungsbereich von 2,25 ± 0,42 mmHg und einer mittleren quadratischen Abweichung von 1,15 ± 0,06 mmHg. Die Schätzung war somit ausreichend genau. Es wurde ein Konkordanzindex von 95,5 % (Vier-Quadranten-Diagramm) bzw. 94,3 % (Polardiagramm) berechnet, was für eine gute Trendfähigkeit spricht.

Die Vorteile des Verfahrens lassen sich unter anderem auf folgende Punkte zurückführen.

Zunächst werden volumenbasierte Messdaten statt zeitbasierter Messdaten verwendet. Insbesondere mithilfe der Kapnographie bzw. Oxigraphie können zahlreiche aussagekräftige Parameter bezüglich des Gasaustauschs überwacht werden. Diese Parameter stellen eine robustere Datenbasis für die Beurteilung des Gasaustauschs als rein zeitbasierte Messdaten bezüglich des ausgeatmeten Atemgasstroms dar.

Des Weiteren konnte gezeigt werden, dass die parallele Auswertung mehrerer Parameter zu einer genaueren Schätzung als die Auswertung eines einzelnen Parameters (z. B. des endtidalen Kohlendioxidpartialdrucks) führt.

Die Verwendung eines Machine-Learning-Moduls bietet zudem den Vorteil, dass nicht lineare, versteckte Informationen im Zusammenhang mit dem pulmonalen Gasaustausch bei jedem Atemzug mit berücksichtigt werden können.

Abschließend wird darauf hingewiesen, dass Begriffe wie "aufweisen", "umfassen", "einschließen", "mit" usw. keine anderen Elemente oder Schritte ausschließen und unbestimmte Artikel wie "ein" oder "eine" keine Vielzahl ausschließen.

Ferner wird darauf hingewiesen, dass Merkmale oder Schritte, die mit Verweis auf eine der vorstehenden Ausführungsformen beschrieben sind, auch in Kombination mit Merkmalen oder Schritten, die mit Verweis auf andere der vorstehenden Ausführungsformen beschrieben sind, verwendet werden können.

Bezugszeichen in den Ansprüchen sind nicht als Beschränkung des Umfangs des durch die Ansprüche definierten Gegenstands zu verstehen.

### Liste der Bezugszeichen

- 1: Beatmungsgerät
- 3: Sensorik
- 5: Messdaten
- 7: Datenverarbeitungsvorrichtung
- 9: Speicher
- 11: Prozessor
- 13: volumenabhängiger Verlauf
- 14: Eingabedaten
- 15: Machine-Learning-Modul
- 17: Ausgabedaten
- 19: Messdatensatz
- 21: Patient
- 23: Beatmungsschritt
- 25: Trainingsdatensatz
- 27: Auswahlmodul
- 29: Zieldaten
- 31: Optimierungsmodul
- 33: mathematische Funktion
- 35: künstliches neuronales Netz
- 37: Eingabeschicht
- 39: Ausgabeschicht
- 41: Zwischenschicht
- 200: Empfangen von Messdaten
- 202: Erzeugen von Eingabedaten
- 204: Eingeben von Eingabedaten
- 206: Ausgeben von Ausgabedaten
- 300: Empfangen von Messdatensätzen
- 302: Erzeugen von Trainingsdatensätzen
- 304: Eingeben von Eingabedaten
- 306: Ausgeben von Ausgabedaten
- 308: Bestimmen einer Abweichung
- 310: Anpassen von Gewichten
- Aw-alv: Wendepunkt (Grenze zwischen Atemwegen und Alveolen)
- I: erste Ausatmungsphase
- II: zweite Ausatmungsphase
- III: dritte Ausatmungsphase
- pCO₂: Kohlendioxidpartialdruck
- pₐCO₂: arterieller Kohlendioxidpartialdruck
- p_{A}CO₂: alveolärer Kohlendioxidpartialdruck
- pₑₜCO₂: endtidaler Kohlendioxidpartialdruck
- p_{E̅}CO₂: gemischt exspiratorischer Kohlendioxidpartialdruck
- V: Atemluftvolumen
- V_{Daw}: Atemwegstotraum
- V_{T}: Atemzugvolumen
- V_{Talv}: alveoläres Tidalvolumen

## Patentansprüche

1. Computerimplementiertes Verfahren zum Schätzen einer Konzentration (pₐCO₂) eines Atemgases im Blut eines Patienten (21), wobei das Verfahren umfasst:
Empfangen (200) von Messdaten (5), die einen volumenabhängigen Verlauf (13) einer Konzentration (pCO₂) des Atemgases in einem vom Patienten (21) ausgeatmeten Atemluftstrom abhängig von einem vom Patienten (21) ausgeatmeten Atemluftvolumen (V) anzeigen;
Erzeugen (202) von Eingabedaten (14) aus den Messdaten (5), wobei die Eingabedaten (14) eine Matrix von Werten für verschiedene Parameter bezüglich des volumenabhängigen Verlaufs (13) umfassen;
Eingeben (204) der Eingabedaten (14) in ein Machine-Learning-Modul (15), das trainiert wurde, um die Eingabedaten (14) in Ausgabedaten (17) umzuwandeln, wobei die Ausgabedaten (17) eine Konzentration (pₐCO₂) des Atemgases im Blut des Patienten (21) anzeigen;
Ausgeben (206) der Ausgabedaten (17) durch das Machine-Learning-Modul (15).

2. Computerimplementiertes Verfahren zum Trainieren eines Machine-Learning-Moduls (15) für ein medizintechnisches Gerät (1), wobei das Verfahren umfasst:
Empfangen (300) mehrerer Messdatensätze (19), die jeweils Messdaten (5) umfassen, die einen volumenabhängigen Verlauf (13) einer Konzentration (pCO₂) eines Atemgases in einem von einem Patienten (21) ausgeatmeten Atemluftstrom abhängig von einem vom Patienten (21) ausgeatmeten Atemluftvolumen (V) anzeigen, wobei die Messdaten (5) verschiedener Messdatensätze (19) zumindest teilweise verschiedenen Patienten (21) zugeordnet sind;
Erzeugen (302) mehrerer Trainingsdatensätze (25) aus den Messdatensätzen (19), wobei jeder Trainingsdatensatz (25) einem der Patienten (21) zugeordnet ist und eine Matrix von Werten für verschiedene Parameter bezüglich des volumenabhängigen Verlaufs (13) umfasst;
Eingeben (304) eines jeden Trainingsdatensatzes (25) als Eingabedaten (14) in das Machine-Learning-Modul (15), das konfiguriert ist, um die Eingabedaten (14) in Ausgabedaten (17) umzuwandeln, wobei die Ausgabedaten (17) eine Konzentration (pₐCO₂) des Atemgases im Blut des jeweiligen Patienten (21) anzeigen;
Ausgeben (306) der Ausgabedaten (17) durch das Machine-Learning-Modul (15);
Bestimmen (308) einer Abweichung der Ausgabedaten (17) von Zieldaten (29), die dem jeweiligen Trainingsdatensatz (25) zugeordnet sind;
Anpassen (310) von Gewichten des Machine-Learning-Moduls (15) in einem Optimierungsverfahren, um die Abweichung zu verringern.

3. Verfahren nach einem der vorhergehenden Ansprüche,
wobei die Messdaten (5) den volumenabhängigen Verlauf (13) bezogen auf einen einzelnen Atemzug des Patienten (21) anzeigen; und/oder
wobei die Messdaten (5) ferner einen dem volumenabhängigen Verlauf (13) zugeordneten positiven endexspiratorischen Druck zum Beatmen des Patienten (21) anzeigen.

4. Verfahren nach einem der vorhergehenden Ansprüche,
wobei eine mathematische Funktion (33), die zumindest einen Abschnitt des volumenabhängigen Verlaufs (13) näherungsweise definiert, unter Verwendung der Messdaten (5) bestimmt wird, wobei mindestens einer der Werte in der Matrix unter Verwendung der mathematischen Funktion (33) berechnet wird.

5. Verfahren nach Anspruch 4,
wobei die Messdaten (5) in mehreren aufeinanderfolgenden Zeitschritten erzeugt wurden und die mathematische Funktion (33) unter Verwendung der Messdaten (5) aus verschiedenen Zeitschritten bestimmt wird; und/oder
wobei die mathematische Funktion (33) gemäß dem Levenberg-Marquardt-Algorithmus bestimmt wird.

6. Verfahren nach einem der vorhergehenden Ansprüche,
wobei die verschiedenen Parameter mindestens einen der folgenden Parameter umfassen: ein bei einem einzelnen Atemzug vom Patienten (21) ausgeatmetes Gesamtvolumen des Atemgases; ein bei einem einzelnen Atemzug vom Patienten (21) ausgeatmetes Gesamtvolumen (V_{T}) einer das Atemgas umfassenden Atemluft; ein Atemminutenvolumen; eine alveoläre Ventilation; einen Atemwegstotraum (V_{Daw}); einen gemischt exspiratorischen Partialdruck (p_{E̅}CO₂) des Atemgases; einen endtidalen Partialdruck (pₑₜCO₂) des Atemgases; einen positiven endexspiratorischen Druck zum Beatmen des Patienten (21); und/oder
wobei der volumenabhängige Verlauf (13) in mindestens drei aufeinanderfolgende charakteristische Ausatmungsphasen (I, II, III) bei einem einzelnen Atemzug des Patienten (21) unterteilt wird, wobei die verschiedenen Parameter mindestens einen der folgenden Parameter umfassen: ein erstes Atemgasvolumen als ein in einer ersten (I) der Ausatmungsphasen (I, II, III) vom Patienten (21) ausgeatmetes Volumen des Atemgases; ein zweites Atemgasvolumen als ein in einer zweiten (II) der Ausatmungsphasen (I, II, III) vom Patienten (21) ausgeatmetes Volumen des Atemgases; ein drittes Atemgasvolumen als ein in einer dritten (III) der Ausatmungsphasen (I, II, III) vom Patienten (21) ausgeatmetes Volumen des Atemgases; eine Steigung des volumenabhängigen Verlaufs (13) in mindestens einer der Ausatmungsphasen (I, II, III), insbesondere in einer letzten (III) der Ausatmungsphasen (I, II, III).

7. Verfahren nach Anspruch 6,
wobei mindestens ein normiertes Atemgasvolumen durch Dividieren eines der drei Atemgasvolumen durch ein bei dem einzelnen Atemzug vom Patienten (21) ausgeamtetes Gesamtvolumen (V_{T}) einer das Atemgas umfassenden Atemluft bestimmt wird, wobei die Parameter das mindestens eine normierte Atemgasvolumen umfassen.

8. Verfahren nach einem der vorhergehenden Ansprüche,
wobei das Machine-Learning-Modul (15) ein künstliches neuronales Netz (35) umfasst.

9. Verfahren nach Anspruch 8,
wobei das künstliche neuronale Netz (35) als ein adaptives Neuro-Fuzzy-Inferenzsystem implementiert ist.

10. Verfahren nach einem der vorhergehenden Ansprüche,
wobei die Messdaten (5) mindestens erste Messdaten und zweite Messdaten umfassen, wobei die ersten Messdaten einen volumenabhängigen Verlauf (13) einer Konzentration (pCO₂) eines ersten Atemgases im Atemluftstrom abhängig vom Atemluftvolumen (V) anzeigen und die zweiten Messdaten einen volumenabhängigen Verlauf (13) einer Konzentration eines zweiten Atemgases im Atemluftstrom abhängig vom Atemluftvolumen (V) anzeigen;
wobei die Ausgabedaten (17) eine Konzentration (pₐCO₂) des ersten Atemgases und eine Konzentration des zweiten Atemgases im Blut des Patienten (21) anzeigen.

11. Verfahren nach Anspruch 10,
wobei die Eingabedaten (14) aus den ersten Messdaten erzeugte erste Eingabedaten und aus den zweiten Messdaten erzeugte zweite Eingabedaten umfassen;
wobei die ersten Eingabedaten in ein erstes künstliches neuronales Netz eingegeben werden und erste Ausgabedaten, die die Konzentration (pₐCO₂) des ersten Atemgases im Blut des Patienten (21) anzeigen, durch das erste künstliche neuronale Netz ausgegeben werden;
wobei die zweiten Eingabedaten in ein zweites künstliches neuronales Netz eingegeben werden und zweite Ausgabedaten, die die Konzentration des zweiten Atemgases im Blut des Patienten (21) anzeigen, durch das zweite künstliche neuronale Netz ausgegeben werden;
wobei die Ausgabedaten (17) die ersten Ausgabedaten und die zweiten Ausgabedaten umfassen.

12. Datenverarbeitungsvorrichtung (7), umfassend einen Prozessor (11), der konfiguriert ist, um mindestens eines der Verfahren nach einem der vorhergehenden Ansprüche auszuführen.

13. Medizintechnisches Gerät (1), umfassend:
eine Sensorik (3) zum Erzeugen von Messdaten (5), die einen volumenabhängigen Verlauf (13) einer Konzentration (pCO₂) eines Atemgases in einem von einem Patienten (21) ausgeatmeten Atemluftstrom abhängig von einem vom Patienten (21) ausgeatmeten Atemluftvolumen (V) anzeigen;
eine Datenverarbeitungsvorrichtung (7) nach Anspruch 12.

14. Computerprogramm, umfassend Befehle, die einen Prozessor (11) beim Ausführen des Computerprogramms durch den Prozessor (11) veranlassen, mindestens eines der Verfahren nach einem der Ansprüche 1 bis 11 auszuführen.

15. Computerlesbares Medium, auf dem das Computerprogramm nach Anspruch 14 gespeichert ist.
